# EUROPÄISCHE PATENTSCHRIFT

(11) **EP 0 542 117 B1**
(45) Veröffentlichungstag und Bekanntmachung des Hinweises auf die Patenterteilung: **13.09.1995**
(21) Anmeldenummer: 92118865.2
(22) Anmeldetag: 04.11.1992
(51) Int. Cl.: C07C 68/02, C07C 69/96

(54) **Verfahren zur Herstellung von Chlorameisensäurearylestern**
Process for the preparation of aryle esters of chloroformic acid
Procédé de préparation des esters aryliques d'acide chloroformique

(30) Priorität: 15.11.1991 DE 4137640
(43) Veröffentlichungstag der Anmeldung: 19.05.1993
(73) Patentinhaber: BASF Aktiengesellschaft, 67063 Ludwigshafen (DE)
(72) Erfinder: Wettling, Thomas, Dr., W-6703 Limburgerhof (DE); Henkelmann, Jochem, Dr., W-6700 Ludwigshafen (DE); Troetsch-Schaller, Irene, Dr., W-6710 Frankenthal (DE); Koehler, Hermann, Dr., W-6719 Bobenheim (DE)

(56) Entgegenhaltungen:
- EP-A- 0 040 741
- US-A- 3 211 774

## Beschreibung

Die Erfindung betrifft ein Verfahren zur Herstellung von Chlorameisensäurearylestern durch Umsetzung von Phenolen mit Phosgen in Gegenwart von organischen Phosphorverbindungen.

Es ist bereits bekannt, Alkohole oder Phenole mit Phosgen zu den entsprechenden Chlorameisensäureestern umzusetzen (Ullmanns Encyklopädie der technischen Chemie, 4. Auflage, Band 9, Seite 381, Verlag Chemie 1975). Während aliphatische Alkohole auch ohne Zusätze mit Phosgen reagieren können, müssen bei der Umsetzung von Phosgen mit Phenolen Zusätze angewendet werden, beispielsweise solche, die in der Lage sind, die freiwerdende Salzsäure zu binden.

So können beispielsweise anorganische Basen, wie wäßrige Natronlauge, eingesetzt werden (DE-A-11 17 598, GB-A-1 200 768). Diese Basen müssen in wenigstens stöchiometrischer Menge eingesetzt werden, was die Wirtschaftlichkeit des Verfahrens belastet und Probleme der Entsorgung aufwirft, da wenigstens stöchiometrische Mengen an anorganischen Salzen entstehen. Weiterhin beeinträchtigt die Arbeitsweise im Zweiphasensystem Wasser/organisches Lösungsmittel die Raum-Zeit-Ausbeute des Verfahrens.

Weiterhin ist der Zusatz von organischen Stickstoffbasen bekannt (DE-A-12 13 419, US 3 211 776). Ihre Verwendung macht es jedoch nötig, die Reaktion in einem organischen Lösungsmittel oder unter erhöhtem Druck im Autoklaven durchzuführen. Auch hierdurch wird die Raum-Zeit-Ausbeute des Verfahrens belastet, bzw. werden Apparaturen zum Arbeiten unter Druck erforderlich. Das aus der zugesetzten Aminbase und dem freiwerdenden Chlorwasserstoff entstehende Hydrochlorid muß aus dem Reaktionsgemisch entfernt werden, beispielsweise durch Waschen der Lösung, Extraktion, Filtration oder Dekantieren, da anderenfalls das entstandene Hydrochlorid bei der folgenden Destillation zu Störungen führt. Zur Vermeidung dieser Komplikationen kann in Gegenwart eines Harzes, das Aminogruppen enthält, gearbeitet werden (US-A-3 211 775), jedoch muß auch dann unter Druck gearbeitet werden. Die zusätzlichen Kosten für diese polymeren Katalysatoren belasten die Wirtschaftlichkeit des Verfahrens. Weiterhin ist das Arbeiten mit Phosgen und dem entstehenden Chlorwasserstoff unter Druck aufwendig.

Weiterhin wurden auch Carbonsäureamide, wie Dimethylformamid, als katalytisch wirksame Zusätze empfohlen (DE-A-21 31 555).

Die US-A 3 211 774 lehrt die Herstellung von Chlorameisensäureestern aus Phenolen und Phosgen in Gegenwart stickstoffhaltiger Verbindungen wie Hexamethylphosphorsäuretriamid und Dimethylformamid.

Weiterhin ist die Verwendung von trisubstituierten Phosphinen, Phosphinoxiden und Phosphoniumsalzen zur Herstellung von aromatischen Chlorameisensäureestern bekannt (DE-A-30 19 526). Diese besitzen eine Aktivität, die zu wünschen übrig läßt.

Der vorliegenden Erfindung lag daher die Aufgabe zugrunde den zuvor genannten Nachteilen abzuhelfen.

Demgemäß wurde ein neues und verbessertes Verfahren zur Herstellung aromatischer Chlorameisensäurearylester der allgemeinen Formel I 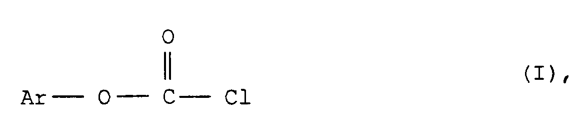
in der
- Ar: ein gegebenenfalls ein- bis fünffach durch C₁- bis C₂₀-Alkyl, C₂- bis C₂₀-Alkenyl, C₃- bis C₈-Cycloalkyl, C₄- bis C₂₀-Alkylcycloalkyl, C₁- bis C₂₀-Halogenalkyl, C₁- bis C₂₀-Alkoxy, C₁- bis C₂₀-Alkylthio, Halogen, Cyano, C₂- bis C₂₀-Alkylcarbonyloxy, C₃- bis C₂₀-Carboalkoxy, Formyl, C₂- bis C₂₀-Dialkylamino, Aryl, Aryloxy, Arylthio, Aroyl, C₇- bis C₂₀-Aralkyl, C₇- bis C₂₀-Aralkoxy, Arylsulfonyl und/oder C₇- bis C₂₀-Aralkylthio und/oder ein- bis zweifach durch Chlorformyl und/oder Nitro substituiertes Aryl
bedeutet, aus Phosgen und Phenolen der allgemeinen Formel II

Ar-OH (II)

,

in der Ar die obengenannten Bedeutungen hat sowie gegebenenfalls ein- bis zweifach Hydroxygruppen trägt, gefunden, welches dadurch gekennzeichnet, daß man die Umsetzung bei einer Temperatur von 60 bis 180°C in Gegenwart organischer Phosphorverbindungen der allgemeinen Formel III 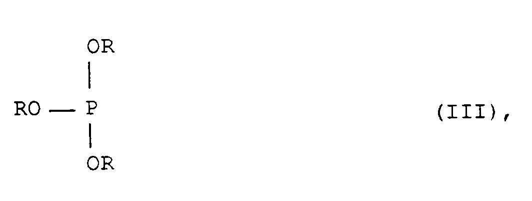
in der R für Aryl oder C₇-C₂₀-Aralkyl steht, durchführt.

Das erfindungsgemäße Verfahren läßt sich wie folgt durchführen:
Das Phenol der allgemeinen Formel II kann in Gegenwart von Katalysatoren bei erhöhter Temperatur mit Phosgen zum Phenylchlorformiaten der allgemeinen Formel I umgesetzt werden.

Das erfindungsgemäße Verfahren kann kontinuierlich oder diskontinuierlich durchgeführt werden. Ein kontinuierliches Verfahren kann beispielsweise im Reaktionsrohr, in einer Rührkesselkaskade, in einem Schlaufenreaktor oder in einer Gegenstromkolonne durchgeführt werden.

Man kann das erfindungsgemäße Verfahren als Flüssigphasenreaktion bei Temperaturen von 60 bis 180°C, bevorzugt von 80 bis 160°C, besonders bevorzugt von 100 bis 140°C und Drücken von 0,01 bis 50 bar, bevorzugt 0,5 bis 5 bar, besonders bevorzugt bei Normaldruck (Atmosphärendruck), vorzugsweise in homogener flüssiger Phase durchführen. Homogene flüssige Phasen sind beispielsweise die Schmelze der Phenole II oder die Phenole II in einem inerten Lösungsmittel.

Die organische Phosphorverbindung kann in einer Menge von beispielsweise 0,1 bis 20 Mol-%, bevorzugt 0,2 bis 10 Mol-%, besonders bevorzugt 0,5 bis 5 Mol-%, bezogen auf jede Hydroxygruppe des Phenols II eingesetzt werden.

Das Molverhältnis von Phosgen zum Phenol II beträgt 1:1 bis 2:1, bevorzugt 1:1 bis 1,5:1, besonders bevorzugt 1:1 bis 1,2:1. Die Verwendung von größeren Überschüssen als 2:1 ist zwar möglich, aber allgemein nicht angezeigt, da sie keine weiteren Vorteile bringt. Die Verwendung von weniger als der stöchiometrischen Menge Phosgen ist möglich, bringt aber im allgemeinen keinen Vorteil, da hierbei nicht umgesetztes Phenol abgetrennt werden muß und die Ausbeute sinkt.

Als Lösungsmittel eignen sich beispielsweise ein aliphatischer oder aromatischer Kohlenwasserstoff, wie Pentan, Hexan, Cyclohexan, Toluol, Xylol oder Benzol, halogenierte Kohlenwasserstoffe, wie Trichlorethan, Chlorbenzol oder Dichlorbenzol oder Ester, wie Ethylacetat oder Butylacetat oder Chlorformiat des entsprechenden Phenols.

In bevorzugter Weise wird entweder ohne Lösungsmittel nur in der Schmelze des Phenols, oder unter Verwendung der z.B. durch Phosgenierung der beschriebenen Phenole entstehenden Chlorformiate als Lösungsmittel gearbeitet. Hierdurch wird eine höhere Raum-Zeit-Ausbeute erzielt als beim Arbeiten in Gegenwart anderer Lösungsmittel, da das Abdestillieren des Lösungsmittels entfällt. Jedoch kann das Arbeiten im Lösungsmittel dann vorteilhaft sein, wenn der Schmelzpunkt des einzusetzenden Phenols oder des entsprechenden Chlorformiats oberhalb der angestrebten Reaktionstemperatur liegt und das Phenol daher, zumindest zu Beginn der Reaktion, ohne Gegenwart des Lösungsmittels nur langsam mit dem Phosgen reagieren würde. Die Gegenwart eines Lösungsmittels kann auch zur Beherrschung und Abführung der Reaktionswärme der exothermen Reaktion günstig sein.

Die Aufarbeitung des Reaktionsgemisches kann z.B. durch Destillation erfolgen. Der Destillationsrückstand enthält die organische Phosphorverbindung, die für eine weitere Umsetzung des erfindungsgemäßen Verfahrens eingesetzt werden kann.

Die Substituenten Ar und R in den Formeln I, II und III haben folgende Bedeutungen:
- Ar: - Aryl, wie Phenyl, 1-Naphthyl, 2-Naphthyl, 1-Anhryl, 2-Anthryl und 9-Anthryl, bevorzugt Phenyl, 1-Naphthyl und 2-Naphthyl, besonders bevorzugt Phenyl,
- ein gegebenenfalls ein- bis fünffach durch einen der folgenden Reste substituiertes Aryl,
- C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C1- bis C4-Alkyl wie Methyl, Ethyl, n-Propyl, iso-Propyl, n-Butyl, iso-Butyl, sec.-Butyl und tert.-Butyl,
- C₂- bis C₂₀-Alkenyl, bevorzugt C₂- bis C₈-Alkenyl wie Vinyl, Allyl, But-2-en-1-yl, But-4-en-1-yl, But-4-en-2-yl, Pent-2-en-1-yl und 2,2-Dimethyl-pent-1-en-1-yl,
- C₃- bis C₈-Cycloalkyl, bevorzugt C₃- bis C₇-Cycloalkyl wie Cyclopropyl, Cyclobutyl, Cyclopentyl, Cyclohexyl, Cycloheptyl und Cyclooctyl, besonders bevorzugt Cyclopentyl und Cyclohexyl,
- C₄- bis C₂₀-Alkyl-cycloalkyl, bevorzugt C₄- bis C₁₂-Cycloalkyl-alkyl wie Cyclopropyl-methyl, Cyclobutyl-methyl, Cyclopentyl-methyl, Cyclohexyl-methyl, Cyclopropyl-ethyl, Cyclobutyl-ethyl, Cyclopentyl-ethyl, Cyclohexyl-ethyl, Cyclopropyl-propyl, Cyclobutyl-propyl, Cyclopentyl-propyl und Cyclohexyl-propyl, besonders bevorzugt Cyclopentyl-methyl, Cyclohexyl-methyl, Cyclopentyl-ethyl und Cyclohexyl-ethyl.
- C₁- bis C₂₀-Halogenalkyl, bevorzugt C₁- bis C₄-Halogenalkyl, besonders bevorzugt C₁- bis C₄-Fluor-, Chlor- und/oder Bromalkyl wie Fluormethyl, Difluormethyl, Trifluormethyl, Chlormethyl, Dichlormethyl, Trichlormethyl, Brommethyl, Dibrommethyl, Tribrommethyl, 1-Chlorethyl, 2-Chlorethyl, 1,1-Dichlorethyl, 2,2-Dichlorethyl, 1,2-Dichlorethyl und Perchlorethyl,
- C₁- bis C₂₀-Alkoxy, bevorzugt C₁- bis C₁₂-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, n-Nonoxy, iso-Nonoxy, n-Decoxy, iso-Decoxy, n-Undecoxy, iso-Undecoxy, n-Dodecoxy und iso-Dodecoxy, besonders bevorzugt C₁- bis C₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.- Hexoxy, n-Heptoxy, iso-Heptoxy, n-Octoxy, iso-Octoxy,
- C₁- bis C₂₀-Alkylthio, bevorzugt C₁- bis C₈-Alkyl wie Methylthio, Ethylthio, n-Propylthio, iso-Propylthio, n-Butylthio, iso-Butylthio, sec.-Butylthio, tert.-Butylthio, n-Pentylthio, iso-Pentylthio, sec.-Pentylthio, neo-Pentylthio, 1,2-Dimethylpropylthio, n-Hexylthio, iso-Hexylthio, sec.-Hexylthio, n-Heptylthio, iso-Heptylthio, n-Octylthio, iso-Octylthio, besonders bevorzugt C₁- bis C₄-Alkyl wie Methylthio, Ethylthio, n-Propylthio, iso-Propylthio, n-Butylthio, iso-Butylthio, sec.-Butyl und tert.-Butylthio,
- Halogen wie Fluor, Chlor, Brom und Jod, bevorzugt Fluor, Chlor und Brom, besonders bevorzugt Chlor und Brom,
- Cyano,
- C₂- bis C₂₀-Alkylcarbonyloxy, bevorzugt C₂- bis C₈-Alkylcarbonyloxy wie Acetyl, Propionyl, Butyryl, Butylcarbamyloxy, Pentylcarbamyloxy, Hexylcarbamyloxy und Heptacarbonyloxy, besonders bevorzugt Acetyl, Propionyl und Butyryl,
- C₃- bis C₂₀-Carboalkoxy, bevorzugt C₃- bis C₈-Carboalkoxy wie Carbomethoxy, Carboethoxy und Carbopropoxy, bevorzugt Carbmethoxy,
- Formyl,
- C₂- bis C₂₀-Dialkylamino, bevorzugt Di-C₁-C₆-alkylamino, bevorzugt Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)mamino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)amino, N-Butyl-N-ethylamino, N-Ethyl-N-(1-methyl-propyl)amino, N-Ethyl-N-(2-methyl-propyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methyl-ethyl)amino, N-(1-Methylethyl)-N-(1-methylpropyl)amino, N-(1-Methylethyl)-N-(2-methylpropyl)amino, N-(1,1-Dimethyethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino, vorzugsweise N,N-Dimethylamino, N,N-Diethylamino und N,N-Dipropylamino, insbesondere N,N-Dimethylamino,
- Aryl, wie Phenyl, 1-Naphthyl, 2-Naphthyl, 1-Anthryl, 2-Anthryl und 9-Anthryl, o-Tolyl, m-Tolyl und p-Tolyl, bevorzugt Phenyl, o-Tolyl, m-Tolyl und p-Tolyl, besonders bevorzugt Phenyl,
- Aryloxy wie Phenoxy, 1-Naphthoxy, 2-Naphthoxy, 1-Anthroxy, 2-Anthroxy, 9-Anthroxy, o-Kresyl, m-Kresyl und p-Kresyl, bevorzugt Phenoxy, o-Kresyl, m-Kresyl und p-Kresyl, besonders bevorzugt Phenoxy, m-Kresyl und o-Kresyl,
- Arylthio wie Phenylthio, 1-Naphthylthio, 2-Naphthylthio, 1-Anthrylthio, 2-Anthrylthio und 9-Anthrylthio, bevorzugt Phenylthio, 1-Naphthylthio und 2-Naphthylthio, besonders bevorzugt Phenylthio,
- Aroyl wie Phenoyl, 1-Naphthoyl, 2-Naphthoyl, 1-Anthroyl, 2-Anthroyl und 9-Anthroyl, bevorzugt Phenoyl, 1-Naphthoyl und 2-Naphthoyl, besonders bevorzugt Phenoyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₇- bis C₂₀-Aralkoxy, bevorzugt C₇- bis C₁₂-Phenylalkoxy wie Benzyloxy, 1-Phenethoxy, 2-Phenethoxy, 1-Phenyl-propoxy, 2-Phenyl-propoxy, 3-Phenyl-propoxy, 1-Phenyl-butoxy, 2-Phenyl-butoxy, 3-Phenyl-butoxy und 4-Phenyl-butoxy, besonders bevorzugt Benzyl, 1-Phenethoxy und 2-Phenethoxy,
- Arylsulfonyl wie Phenylsulfonyl, 1-Naphthylsulfonyl, 2-Naphthylsulfonyl, 1-Anthrylsulfonyl, 2-Anthrylsulfonyl und 9-Anthrylsulfonyl, bevorzugt Phenylsulfonyl, 1-Naphthylsulfonyl und 2-Naphthylsulfonyl, besonders bevorzugt Phenylsulfonyl,
- C₇- bis C₂₀-Aralkylthio bevorzugt C₇- bis C₁₂-Phenylalkylthio wie Benzylthio, 1-Phenethylthio, 2-Phenethylthio, 1-Phenyl-propylthio, 2-Phenyl-propylthio, 3-Phenyl-propylthio, 1-Phenyl-butylthio, 2-Phenyl-butylthio, 3-Phenyl-butylthio und 4-Phenyl-butylthio, besonders bevorzugt Benzylthio, 1-Phenethylthio und 2-Phenethylthio,
- und/oder ein gegebenenfalls ein- bis zweifach durch einen der folgenden Reste substituiertes Aryl,
- Chlorformyl
- Nitro - sowie im Falle von den Verbindungen II noch zusätzlich ein oder zwei Hydroxyfunktionen.
- R: steht für
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl.
Als Beispiele für die organischen Phosphorverbindungen der Formel (III) sei beispielsweise Triphenylphosphit, genannt. Beispiele für einzelne erfindungsgemäß einsetzbare Phenole II sind:
Phenol, o-Kresol, m-Kresol, p-Kresol, Xylenole, 4-Chlorphenol, Hydrochinon, Resorcin, 1-Naphthol, 2-Naphthol, 1,5-Dihydroxynaphthalin, 4,4'-Methylenbisphenol, 1,1-Bis-(4-hydroxyphenyl)-ethan, 2,2-Bis-(4-hydroxyphenyl)-propan, 4,4'-Cyclohexyliden-bisphenol, 4,4'-Dihydroxydiphenylether, 4,4'-Dihydroxydiphenylsulfid, 4,4'-Dihydroxydiphenylsulfon, 4,4'-Dihydroxydiphenyl-N-methylamin, 3-Hydroxybenzophenon und 4-Hydroxybenzophenon.

Als Chlorameisensäurearylester, die nach dem erfindungsgemäßen Verfahren hergestellt werden können, seien beispielsweise genannt:
Chlorameisensäurephenylester, -o-kresylester, -m-kresylester, -p-kresylester, -dimethylphenylester, -4-chlorphenylester, -naphthylester, Hydrochinon-, Resorcin-, 1,5-Dihydroxynaphthalin-bis-chloroformiat, 4,4'-Methylenbisphenyl-, 1,1-Bis-(4-hydroxyphenyl)-ethan-, 2,2-Bis-(4-hydroxyphenyl)-propan-, 4,4'-Cyclohexyliden-bisphenyl-, 4,4'-Dihydroxydiphenylether-bis-chloroformat und Benzophenon-4-chlorformiat.

Die Chlorameisensäurearylester, insbesondere der Chlorameisensäurephenylester, werden nach dem erfindungsgemäßen Verfahren in hoher Reinheit erhalten. Sie können daher für viele Zwecke als Rohprodukte weiterverwendet werden. Selbstverständlich können sie in bekannter Weise, beispielsweise durch Destillation oder Umkristallisation, auch weiter gereinigt werden.

Chlorameisensäurearylester sind wertvolle Zwischenprodukte, beispielsweise bei der Herstellung von Farbstoffen, wie Sirius-Farbstoffe (DE-A-23 25 088, Ullmanns Enzyklopädie der technischen Chemie, Band 4, Seite 105, 108 und 109, Urban und Schwarzenberg, 3. Auflage, Berlin-München., 1953), zur Herstellung von Polycar-bonatkunststoffen, Pflanzenschutzmitteln und zur Herstellung von Bakteriziden. Diese Verwendungszwecke sind dem Fachmann bekannt und beispielsweise beschrieben in DE-A-21 31 555, DE-A-12 13 419 und Ullmanns Enzyklopädie der technischen Chemie, 4. Auflage, Band 9, Seite 383, Verlag Chemie 1975.

### Beispiele

### Vergleichsbeispiele

### Beispiel 1

### (Durchführung in Analogie zu DE-A-21 31 555, Beispiel 2)

In einem Rundkolben, der mit einem Rührer, einem Thermometer, einem Phosgeneinleitungsrohr, einem beheizbaren Tropftrichter und einem mit Trockeneis und Aceton gefüllten Kühler ausgestattet ist, wird ein Gemisch aus 100 g Phenylchlorformiat und 3,7 g (0,05 mol) N,N-Dimethylformamid auf 120°C erhitzt und während 4 Stunden 112 g (1,13 mol) Phosgen zusammen mit 94 g (1 mol) Phenol zudosiert. Nach 1 Stunde Nachreaktionszeit bei gleicher Temperatur entfernt man den Rückflußkühler und bläst überschüssiges Phosgen durch Einleiten von Stickstoff aus dem Reaktionsgemisch aus. Man erhält einen schwarzen Austrag, aus dem Reaktionsnebenprodukte in Form eines schwarzen Belags an der Kolbeninnenseite, ausfallen.

Das Rohprodukt besitzt einen Phenylchlorformiatgehalt von 97,9 % und einen Diphenylcarbonatgehalt von 1,8 % (GC-Analyse). Man destilliert den Austrag, wobei das Phenylchlorformiat bei etwa 85°C und 20 Torr übergeht. Das destillierte Produkt zeigt bei gaschromatographischer Untersuchung noch deutlich erkennbare Spuren von Verunreinigungen, die sich ohne beträchtlichen Aufwand bei der Destillation nicht abtrennen lassen.

### Beispiel 2

### (Durchführung in Analogie zu DE-A-30 19 526, Beispiel 4)

In einer Apparatur wie in Beispiel 1 beschrieben, legt man 100 g Phenylchlorformiat und 2,6 g (0,01 mol) Triphenylphosphin vor. Bei einer Reaktionstemperatur von 120°C werden während 4 Stunden 113 g (1,13 mol) Phosgen und 94 g (1 mol) Phenol zudosiert. Nach 1 Stunde Nachreaktion wird überschüssiges Phosgen ausgeblasen und man erhält ein klares, hellgelbes Rohprodukt mit einem Phenylchlorformiatgehalt von 97,6 % und einem Diphenylcarbonatgehalt von 1,7 % (GC-Analyse). Man destilliert den Austrag und erhält im Destillationsrückstand Feststoffanfall.

### Beispiel 3

Man geht vor wie in Beispiel 6 beschrieben, verwendet jedoch 9,0 g (0,05 mol) Hexamethylphosphorsäuretriamid als Katalysator und erhält nach 4 Stunden Reaktionszeit und 1 Stunde Nachreaktionszeit ein hellgelbes, klares Rohprodukt mit einem Phenylchlorformiatgehalt von 95,7 % und einem Diphenylcarbonatgehalt von 2,5 % (GC-Analyse).

### Beispiel 4

Man geht vor wie in Beispiel 6 beschrieben, verwendet jedoch den Destillationsrückstand der Reaktion aus Beispiel 3 als Katalysator und erhält nach 2 Stunden Reaktionszeit und 1 Stunde Nachreaktionszeit ein gelbes, klares Rohprodukt mit einem Phenylchlorformiatgehalt von 89,2 % und einem Diphenylcarbonatgehalt von 6,9 % (GC-Analyse).

### Beispiel 5

Man geht vor wie in Beispiel 6 beschrieben, verwendet jedoch 8,2 g (0,05 mol) Hexamethyltriaminophosphin als Katalysator und erhält nach 2,5 Stunden Reaktionszeit und 1 Stunde Nachreaktionszeit ein dunkles Rohprodukt mit einem Phenylchlorformiatgehalt von 84,5 % und einem Diphenylcarbonatgehalt von 1,3 % (GC-Analyse).

### Erfindungsgemäße Beispiele:

### Beispiel 6

In einer Apparatur und in Analogie zur Vorgehensweise wie in Beispiel 1 werden 94 g (1 mol) Phenol in Gegenwart von 3,1 g (0,01 mol)Phosphorigsäuretriphenylester während 4 Stunden bei 120°C mit Phosgen umgesetzt. Man erhält ein klares, hellgelbes Rohprodukt mit einem Phenylchlorformiatgehalt von 98,3 % und einem Diphenylcarbonatgehalt von 1,4 % (GC-Analyse). Nach der Destillation erhält man ein wasserklares Phenylchlorformiat, das nach GC-Analyse keine Verunreinigungen enthält.

### Beispiel 7

Man geht vor wie in Beispiel 6 beschrieben, verwendet jedoch 6,2 g (0,02 mol) Phosphorigsäuretriphenylester als Katalysator und erhält nach nur 1,5 Stunden Reaktionszeit und 1 Stunde Nachreaktionszeit ein hellgelbes, klares Rohprodukt mit einem Phenylchlorformiatgehalt von 98,5 % und einem Diphenylcarbonatgehalt von 1,2 % (GC-Analyse).

### Beispiel 8

Man geht vor wie in Beispiel 7 beschrieben, legt jedoch kein Phenylchlorformiat und nur 6,2 g (0,02 Mol) Phosphorigsäuretriphenylester vor. Nach 5,5 Stunden Reaktionszeit erhält man ein klares, gelbes Rohprodukt mit einem Phenylchlorformiatgehalt von 97,6 % und einem Diphenylcarbonatgehalt von 1,9 % (GC-Analyse).

### Beispiel 9

Man geht vor wie in Beispiel 7 beschrieben, legt jedoch nur 10 g Phenylchlorformiat und 6,2 g (0,02 mol) Phosphorigsäuretriphenylester vor. Nach 4,5 Stunden Reaktionszeit erhält man ein klares, hellgelbes Rohprodukt mit einem Phenylchlorformiatgehalt von 97,8 % und einem Diphenylcarbonatgehalt von 1,5 % (GC-Analyse).

### Beispiel 10

Man geht vor wie in Beispiel 7 beschrieben, legt jedoch 10 g Phenol und 6,2 g (0,02 mol) Phosphorigsäuretriphenylester vor. Nach 2,5 Stunden Reaktionszeit erhält man ein klares, hellgelbes Rohprodukt mit einem Phenylchlorformiatgehalt von 95,5 % und einem Diphenylcarbonatgehalt von 3,8 % (GC-Analyse).

### Beispiel 11

In einer Apparatur wie in Beispiel 1 beschrieben, legt man 22 g (0,2 mol) m-Kresol und 6,3 g (0,02 mol) Triphenylphosphit vor. Bei einer Reaktionstemperatur von 120°C werden während 4 Stunden 194 g (1,8 mol) m-Kresol 11,3 g (0,035 mol) Triphenylphosphit und 220 g (2,2 mol) Phosgen gleichzeitig zudosiert. Nach 1 Stunde Nachreaktion wird überschüssiges Phosgen ausgeblasen und man erhält ein klares, braunes Rohprodukt mit einem m-Kresylchlorformiatgehalt von 91 % und einem Carbonatgehalt von 1,4 % (GC-Analyse).

### Beispiel 12

In einer kontinuierlich betriebenen Apparatur bestehend aus einer Dosiereinrichtung, einem Hauptreaktor mit Rührer, Phosgeneinleitungsrohr und Überlauf, weiterhin einem Nachreaktor mit Rührer und einer kontinuierlichen Strippkolonne wird 157 g (1 mol) Phenylchlorformiat und 6,3 g (0,02 mol) Triphenylphosphit vorgelegt. Die Temperatur wird sowohl im Haupt- als auch im Nachreaktor auf 125°C eingestellt. Die automatisierte Dosiereinrichtung tropft parallel 1 mol/h Phenol mit 0,02 mol darin gelöstem Triphenylphosphit zu und leitet soviel Phosgen im Überschuß ein (ca. 1,2 mol/h), daß die Reaktionstemperatur konstant bleibt.

Nach einer Anfahrphase erhält man aus der kontinuierlich betriebenen Apparatur ein Rohprodukt mit einem Phenylchlorformiatgehalt von 98.6 % und einem Diphenylcarbonatgehalt von 1,2 % (GC-Analyse).

## Patentansprüche

1. Verfahren zur Herstellung aromatischer Chlorameisensäurealkylester der allgemeinen Formel I 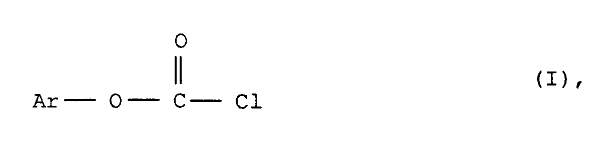 in der
Ar ein gegebenenfalls ein- bis fünffach durch C₁- bis C₂₀-Alkyl, C₂- bis C₂₀-Alkenyl, C₃- bis C₈-Cycloalkyl, C₄- bis C₂₀-Alkyl-cycloalkyl, C₁- bis C₂₀-Halogenalkyl, C₁-bis C₂₀-Alkoxy, C₁- bis C₂₀-Alkylthio, Halogen, Cyano, C₂- bis C₂₀-Alkylcarbonyloxy, C₃- bis C₂₀-Carboalkoxy, Formyl, C₂- bis C₂₀-Dialkylamino, Aryl, Aryloxy, Arylthio, Aroyl, C₇- bis C₂₀-Aralkyl, C₇- bis C₂₀-Aralkoxy, Arylsulfonyl und/oder C₇- bis C₂₀-Aralkylthio und/oder ein- bis zweifach durch Chlorformyl und/oder Nitro substituiertes Aryl
bedeutet, aus Phosgen und Phenolen der allgemeinen Formel II
Ar-OH (II)
,
in der Ar die obengenannten Bedeutungen hat sowie gegebenenfalls ein- bis zweifach Hydroxygruppen trägt, dadurch gekennzeichnet, daß man die Umsetzung bei einer Temperatur von 60 bis 180°C in Gegenwart organischer Phosphorverbindungen der allgemeinen Formel III 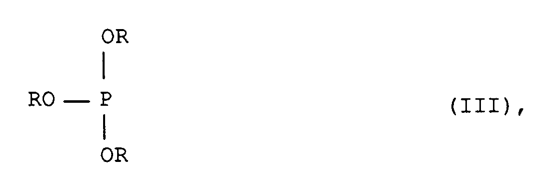 in der R für Aryl oder C₇-C₂₀-Aralkyl steht, durchführt.

2. Verfahren nach Anspruch 1, dadurch gekennzeichnet, daß man als organische Phosphorverbindung Triphenylphosphit verwendet.

3. Verfahren nach Anspruch 1, dadurch gekennzeichnet, daß man die organische Phosphorverbindungen III in einer Menge von 0,1 bis 20 Mol-% pro Äquivalent organische Hydroxylgruppe im Phenol verwendet.

4. Verfahren nach Anspruch 1, dadurch gekennzeichnet, daß man bei einer Temperatur zwischen 80 und 160°C arbeitet.

5. Verfahren nach Anspruch 1, dadurch gekennzeichnet, daß man in Gegenwart eines inerten Lösungsmittels arbeitet.

6. Verfahren nach Anspruch 1, dadurch gekennzeichnet, daß man als inertes Lösungsmittel den zu erzeugenden aromatischen Chlorameisensäureester verwendet.

## Claims

1. A process for preparing aromatic esters of chloroformic acid of the general formula I 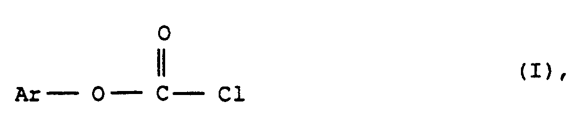 where
Ar is aryl which is unsubstituted or substituted once to five times by C₁-C₂₀-alkyl, C₂-C₂₀-alkenyl, C₃-C₈-cycloalkyl, C₄-C₂₀-alkyl-cycloalkyl, C₁-C₂₀-haloalkyl, C₁-C₂₀-alkoxy, C₁-C₂₀-alkylthio, halogen, cyano, C₂-C₂₀-alkylcarbonyloxy, C₃-C₂₀-carboalkoxy, formyl, C₂-C₂₀-dialkylamino, aryl, aryloxy, arylthio, aroyl, C₇-C₂₀-aralkyl, C₇-C₂₀-aralkoxy, arylsulfonyl and/or C₇-C₂₀-aralkylthio and/or once or twice by chloroformyl and/or nitro, from phosgene and phenols of the general formula II
Ar-OH (II)
,
where Ar has the abovementioned meanings and may carry once or twice hydroxyl groups, wherein the reaction is carried out at from 60 to 180°C in the presence of organic phosphorus compounds of the general formula III 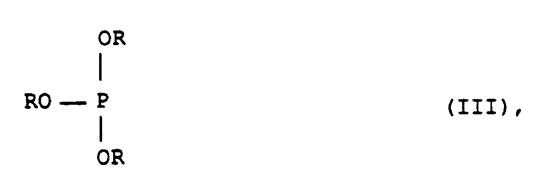 where R is aryl or C₇-C₂₀-aralkyl.

2. A process as claimed in claim 1, wherein triphenyl phosphite is used as organic phosphorus compound.

3. A process as claimed in claim 1, wherein the organic phosphorus compounds III are used in an amount of from 0.1 to 20 mol % per equivalent organic hydroxl group in the phenol.

4. A process as claimed in claim 1, wherein the temperature is from 80 to 160°C.

5. A process as claimed in claim 1, wherein an inert solvent is present.

6. A process as claimed in claim 1, wherein the aromatic ester of chloroformic acid which is to be produced is used as inert solvent.

## Revendications

1. Procédé de préparation de chloroformiates d'alkyle aromatiques de la formule générale I 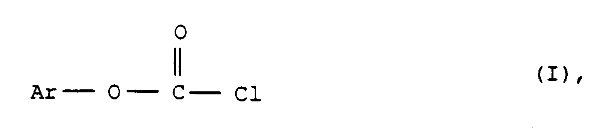 dans laquelle
Ar représente un radical aryle éventuellement substitué de une à cinq fois par des radicaux alkyle en C₁ à C₂₀, alcényle en C₂ à C₂₀, cycloalkyle en C₃ à C₈, alkyl-cycloalkyle en C₄ à C₂₀, halogénoalkyle en C₁ à C₂₀, alcoxy en C₁ à C₂₀, alkylthio en C₁ à C₂₀, éventuellement de une à cinq fois substitué par des halogènes, des radicaux cyano, alkylcarbonyloxy en C₂ à C₂₀, carbalcoxy en C₃ à C₂₀, formyle, dialkylamino en C₂ à C₂₀, aryle, aryloxy, arylthio, aroyle, aralkyle en C₇ à C₂₀, aralcoxy en C₇ à C₂₀, arylsulfonyle et/ou aralkylthio en C₇ à C₂₀ et/ou éventuellement substitué une à deux fois par des radicaux chloroformyle et/ou nitro,
au départ du phosgène et de phénols de la formule générale II
Ar-OH (II),
dans laquelle Ar possède les significations qui lui ont été attribuées ci-dessus, et porte éventuellement un à deux radicaux hydroxyle, caractérisé en ce que l'on entreprend la réaction à une température de 60 à 180°C, en présence de composés organiques du phosphore de la formule générale III 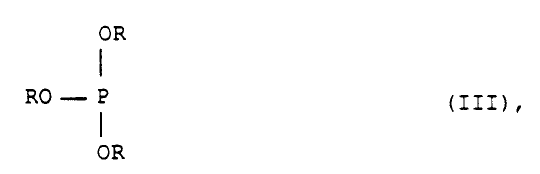 dans laquelle R représente un radical aryle et/ou aralkyle en C₇ à C₂₀.

2. Procédé suivant la revendication 1, caractérisé en ce que l'on utilise le phosphite de triphényle à titre de composé organique du phosphore.

3. Procédé suivant la revendication 1, caractérisé en ce que l'on utilise les composés organiques du phosphore III en une proportion de 0,1 à 20% molaires par équivalent de radicaux hydroxyle organique dans le phénol.

4. Procédé suivant la revendication 1, caractérisé en ce que l'on travaille à une température comprise entre 80 et 160°C.

5. Procédé suivant la revendication 1, caractérisé en ce que l'on travaille en présence d'un solvant inerte.

6. Procédé suivant la revendication 1, caractérisé en ce que l'on utilise l'ester de l'acide chloroformique aromatique à obtenir à titre de solvant.
